Europäisches Patentamt

(19) **European Patent Office**

Office européen des brevets

(11) Numéro de publication: **0 027 088**
**B1**

(12) **FASCICULE DE BREVET EUROPEEN**

(45) Date de publication du fascicule du brevet: **14.12.83**

(51) Int. Cl.³: **A 61 K 39/35,**
**A 61 K 39/36**

(21) Numéro de dépôt: **80401424.9**

(22) Date de dépôt: **06.10.80**

(54) Composition allergénique et procédé pour sa préparation.

(30) Priorité: **08.10.79 FR 7924948**

(43) Date de publication de la demande:
**15.04.81 Bulletin 81/15**

(45) Mention de la délivrance du brevet:
**14.12.83 Bulletin 83/50**

(84) Etats contractants désignés:
**BE CH DE GB IT LI NL SE**

(56) Documents cités:
**US - A - 4 163 778**

BIOCHEMISTRY, vol. 3, no. 3, mars 1964 US
T.P. KING et al.: "Isolation and Characterization
of Allergens from Ragweed Pollen. II", pages
458-468

BIOCHEMISTRY, vol. 8, no. 3, mars 1969 US
B.J. UNDERDOWN et al.: "Isolation and
Characterization of an Allergen from Short
Ragweed Pollen", pages 980-989

(73) Titulaire: **INSTITUT PASTEUR**
**25-28, rue du Docteur Roux**
**F-75724 Paris Cedex 15 (FR)**

(72) Inventeur: **Relyveld, Edgar Hans**
**3, Place Stéfanik**
**F-75016 Paris (FR)**
Inventeur: **Henocq, Emile**
**211, rue de Vaugirard**
**F-75015 Paris (FR)**

(74) Mandataire: **Coutel, Jean-Claude**
**Cabinet AYMARD & COUTEL 20, rue Vignon**
**F-75009 Paris (FR)**

(56) Documents cités:
**CHEMICAL ABSTRACTS, vol. 56, no. 9, 30 avril
1962, col. 10764, réf. no. 10764c Columbus,
Ohio, US H. PALMSTIERNA: "The purification of
allergens. I. Phleum pratense allergen"**

**CHEMICAL ABSTRACTS, vol. 57, no. 8, 15
octobre 1962, col. 10170, réf. no. 10170a
Columbus, Ohio, US T.P. KING et al.: "Isolation
studies of allergens from ragweed pollen"**

Courier Press, Leamington Spa, England.

**0 027 088**

(58) Documents cités:

CHEMICAL ABSTRACTS, vol. 60, no. 12, 8 juin 1964, col. 14989, réf. no. 14989b Columbus, Ohio, US K.C. ROBBINS et al.: "Isolation of nondialyzable allergens and antigens from low ragweed pollen"

THE JOURNAL OF IMMUNOLOGY, vol. 115 no. 1, juillet 1975 US M. ROEBBER et al.: "An Improved Procedure for Isolation of Ragweed Pollen Allergen Ra5", pages 303-304

CHEMICAL ABSTRACTS, vol. 63, no. 8 11 octobre 1965, col. 10487, réf. no. 10487b Columbus, Ohio US P. JOHNSON et al.: "The isolation and characterization of allergens from the pollen of rye grass (Lolium perenne)"

CHEMICAL ABSTRACTS, vol. 84, no. 1,5 janvier 1976, page 290, réf. no. 3173d Columbus, Ohio, US W.G. HARRIS: "Allergens of Schistosoma mannori. II. Separation by Sephadex G-200 and ion-exchange chromatography"

CHEMICAL ABSTRACTS, vol. 78, no. 17, 30 avril 1973, page 316, réf. no. 109129k Columbus, Ohio, US W.G. HARRIS: "Allergens of Schistosoma mansoni. I. Initial separation and comparative assay"

## Composition allergénique et procédé pour sa préparation

L'invention concerne la préparation d'allgergènes, à laquelle elle apporte un perfectionnement permettant l'obtention de compositions allergéniques améliorées; ces dernières font également partie de l'invention.

L'allergologie a pris une place importante dans la thérapeutique au cours des dernières décennies, et son rôle va en s'amplifiant; non seulement décèle-t-elle le caractère allergique dans diverses affections anciennes, mais encore est-elle appelée à remédier aux atteintes de ce type, que multiplie la pollution moderne. Aussi, un besoin de bons agents hyposensibilisants existe toujours, malgré la présence sur le marché d'allergènes bien connus, extraits de différentes matières, notamment: de pollens, farines, poussières de maison, poils, kapok, plumes, moisissures, etc. Bien que plusieurs préparations, et en particulier des allergènes-retard adsorbés sur des supports minéraux, tels que des gels à base d'alumine par exemple, donnent d'excellents résultats, l'inocuité et la constance d'activité de certains d'entre eux laissent souvent à désirer. La durée d'utilisation de plusieurs extraits allergéniques, disponibles à l'heure actuelle, est donc limitée dans le temps.

La présente invention apporte un perfectionnement qui rend possible l'obtention d'allergènes beaucoup plus stables, plus actifs, ne provoquant pas des réactions secondaires, dans l'organisme auquel on les injecte. Les allergènes perfectionnés, produits suivant cette invention, ont une durée d'utilisation considérablement prolongée et ils se prêtent particulièrement bien à la préparation de la forme adsorbée sur un gel minéral.

L'invention résulte de la mise en lumière d'un phénomène qui était ignoré, dans cette technique, jusqu'à présent: la présence de diverses enzymes dans les extraits allergéniques n'était pas considérée comme défavorable, et l'on a même proposé de la mettre à profit pour classer ou apprécier l'activité (GLEICH G. J. et coll. "Allergy and Clinical Immunology" — Excerpta Medical, Amsterdam, 1977, pages 184 et 213). D'autre part, selon l'article dans BIOCHEMISTRY, Vol. No. 3, Mars 1964, page 459, il n'y a pas de rapport autre l'activité allergénique et celle das enzymes présentes. Or, l'Institut Pasteur a trouvé qu'au moins certaines de ces enzymes dégradent des protéines constitutives des allergènes. Ainsi, de façon imprévue, les demandeurs ont déterminé la cause de l'instabilité des extraits allergéniques; ils ont trouvé également que les enzymes, responsables de l'attaque des protéines utiles, ainsi que d'autres impuretés, gênent l'adsorption des allergènes sur des adjuvants minéraux. En outre, ces ou certaines de ces enzymes peuvent être la cause de la production d'anticorps, par une réaction de l'organisme contre les impuretés qu'elles constituent dans l'allergène injecté.

Le procédé perfectionné, suivant l'invention, comprend donc l'élimination des enzymes présentes de l'extrait aqueux d'un allergène, aussitôt que possible après la préparation de cet extrait. Il s'agit, en effet, de laisser le moins de temps possible à l'attaque des protéines utiles par les protéases du milieu en présence.

Ainsi, le procédé suivant l'invention, qui comprend la préparation d'un extrait aqueux d'allergène, est caractérisé en ce que l'on élimine de cet extrait des substances ne présentant pas l'activité allergénique voulue. Dans une forme d'exécution particulière, on laisse dans la solution seulement les substances allergéniquement actives dont les masses moléculaires vont d'environ 10 000 à 50 000, et surtout de 14 000 à 45 000 (par précipitation).

Dans la pratique de l'invention, il est d'ailleurs souhaitable d'effectuer l'opération sus-indiquée sur un extrait déjà débarrassé de différentes autres impuretés; cela se fait, comme connu, par la précipitation et redissolution des protéines de l'extrait.

L'élimination des fractions inactives, notamment celles dont les masses moléculaires sontr inférieures à 10 000 ou à 14 000 et supérieures à 55 000 ou à 45 000, conformément à l'invention, peut être réalisée par tous moyens appropriés, bien connus dans l'art, par exemple des procédés chromatographiques, précipitations fractionnées, électrophorèse, etc. La filtration sur gel rend, dans cette voie, de grands services, aussi décrit-on ci-après, à titre d'exemple non limitatif, le fractionnement d'un extrait de pollen par tamisage moléculaire.

Les méthods d'extraction de protéine de diverses matières, notamment en vue de la préparation de compositions allergéniques, sont connus, il n'y a donc pas lieu de les décrire ici. On rappelle seulement, à titre d'exemple, un mode opératoire qui convient particulièrement bien et qui a fait l'objet de publications comme celle du brevet français no. 1 604 135. Cette méthode consiste à traiter 100 g de matière, notamment de pollens, par 1 litre d'une solution de $Na_2HPO_4.12H_2O$ à 25 g/l, renfermant 1/10 000 de merthiolate. Après 24 heures d'agitation à + 4°C, la solution est séparée du solide par centrifugation. L'extrait brut, ainsi obtenu, est purifié par précipitation saline, qui consiste à ajouter 604 g de sulfate d'ammonium, cristallisé, à 1 litre de cet extrait, et à laisser en contact, sous agitation, pendant 3 h à +4°C. Le précipité formé est alors séparé par centrifugation et redissous dans une solution de phosphate disodique à 25 g/l contenant 1/10 000 de merthiolate. La solution obtenue est dialysée contre une solution fraîche de phosphate disodique à 25 g/l, toujours additionné de merthiolate.

C'est sur un extrait, c'est-à-dire une solution préparée comme indiqué ci-dessus, à partir du pollen de phléole, qu'ont été effectuées les opérations exposées dans la suite de la présente description.

Cette solution est d'abord soumise à un fractionnement par tamisage moléculaire. Pour cela, on utilise une colonne chromatographique de 35 mm de diamètre et 560 mm de haut; elle est chargée de Sephadex G—100 dont le domaine de fractionnement possible s'étend sur les masses moléculaires de 4 000 à 150 000. Le fractionnement est conduit avec un éluant constitué par une solution de phosphate disodique à 25 g de $Na_2HPO_4.12H_2O$ par litre, renfermant 0,9% de NaCl et 1/10 000 de merthiolate. On opère sur des portions de 5 ml d'extrait, chacune d'elles étant suivie d'un passage du tampon d'élution. On recueille des fractions de 10 ml sur lesquelles on détermine:

la masse moléculaire de la substance, dissoute,

la présence d'enzymes et

la réaction sur la peau.

D'autre part, un fractionnement semblable, et les déterminations sus-indiquées, sont effectués sur un extrait du pollen de phléole dont le phosphate disodique, de même concentration, mais non encore purifié par précipitation au sulfate d'ammonium: cette solution est appelée extrait brut dans la suite de la description.

Les résultats de ces essais sont rapportés dans les graphiques et tableaux ci-joints.

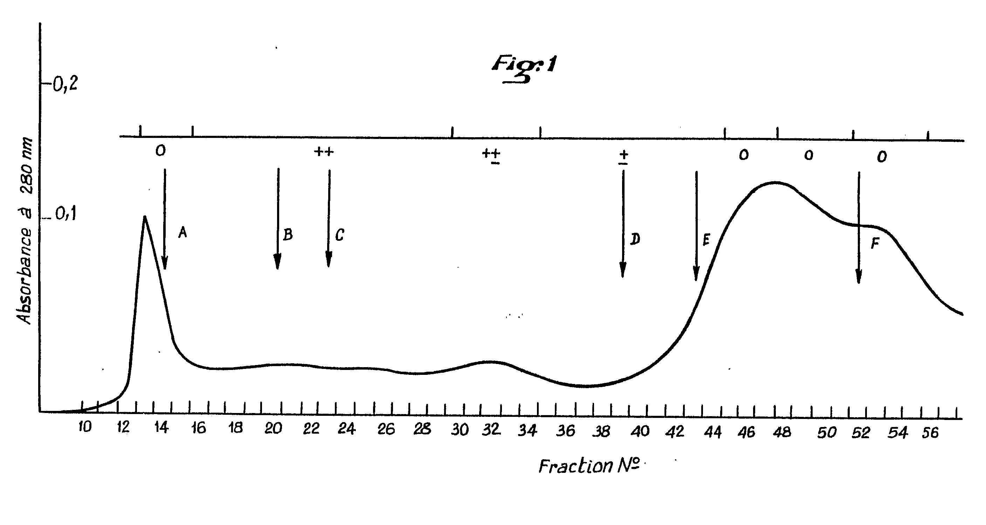

Sur la figure 1, on a tracé la courbe d'élution de l'extrait brut du pollen de phléole: les numéros des fractions de 10 ml sont portés en abscisses, tandis que les ordonnées indiquent l'absorbance à 280 nm. En haut du graphique, sur une ligne parallèle aux abscisses, on note les réactions sur la peau des fractions mélangées depuis la 13 ème jusqu'à la 55 ème fraction. Les lettres A à F désignent les repères de référence de substances à masses moléculaires connues:

A — Bleu de dextran     masse mol. 2 000 000
B — Albumine     masse mol.     65 000
C — Ovalbumine     masse mol.     45 000
D — Lysozyme     masse mol.     14 600
E — Bacitracine     masse mol.     1 450
F — DNP éthanolamine  masse mol.       227

La réaction del apeau est déterminée par la méthode connue de piqûre ("prick"), qui consiste à mettre une goutte de liquide sur la peau et piquer à travers cette goutte avec une aiguille; après 20 minutes environ, un sujet allergique donne une réaction positive, notée +, c'est-à-dire une papule et un érythème. Un seul + signifie que la papule s'étend sur un diamètre moyen de 5 mm; le nombre des + indique le multiple des 5 mm observés.

Dans le cas de l'extrait brut de la figure 1, on constate une réaction de la peau pour les fractions réunies n° 16 à 29, (++), 30 à 34 (+±) et 35 à 44 (±): il n'y a pas de réaction avant la fraction 15, ni après la fraction 45, ce qui signifie qu'en dehors des fractions 16 à 44, il n'y a plus de produit intéressant en tant qu'allergène. Le domaine utile va donc du n° 16 au n° 44.

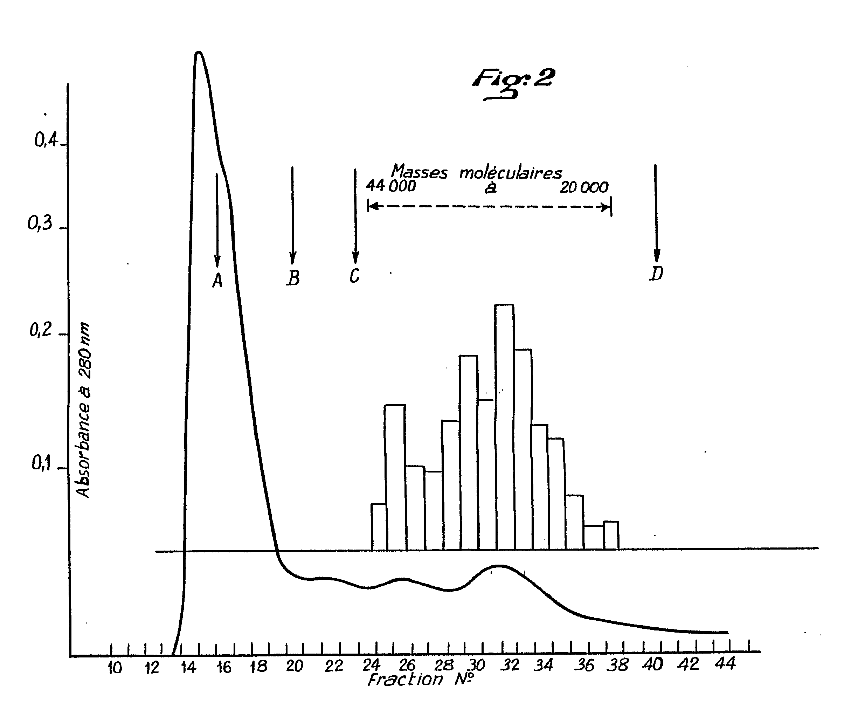

Sur la figure 2, on voit le diagramme d'élution, analogue à celui de la figure 1, mais appliqué à l'extrait du pollen de phléole préalablement purifié par précipitation au sulfate d'ammonium et redissolution dans une solution de phosphate disodique; ce sont donc presque uniquement les protéines qui sont ainsi soumises à la séparation par tamisage moléculaire. Sur ce graphique, la série de rectangles verticaux représentent les seules fractions intéressantes qui donnent des réactions + à +++ sur la peau d'allergiques, déterminées par la méthode de piqûre. Les hauteurs des rectangles sont proportionnelles au diamètre des papules formées sur la peau: 25 mm de hauteur correspondent à 5 mm de diamètre de papule. Le domaine utile comprend les fractions n° 25 à 38, correspondant sensiblement aux masses moléculaires de 44 000 à 20 000. On retrouve donc ici les indications fournies par l'extrait brut de la figure 1, avec cependant un resserrement du domaine utile.

Conformément à l'invention, dans le cas présent, donné à titre d'exemple, on recueille seulement les fractions n° 25 à 38 pour la préparation de la composition allergénique, tandis que les autres fractions sont rejetées, contrairement á ce qui se pratiquait antérieurement.

La preuve que les fractions 25 à 38 retenues ne contiennent pratiquement pas d'enzymes est apportée par des déterminations effectuées par la méthode très pratique, connue sous le nom de système "API ZYM". Cette méthode consiste à introduire dans une série de 20 cupules, dont le fond est constitué d'un support contenant le substrat enzymatique avec son tampon, une petite quantité de liquide à étudier et à faire réagir, après incubation, ce liquide avec deux réactifs, le tris (hydroxy-méthyl)-amino-méthane et le bleu rapid BB. La présence d'enzymes se manifeste par la coloration qui apparaît dans les cupules et que l'on note sur une échelle de 1 à 5, ce dernier chiffre correspondant au maximum d'intensité. A l'aide de ce système, certains auteurs ont pu trouver la présence de nombreuses enzymes dans des extraits de pollen de graminées; ainsi, Jean Bousquet et col. ont fait des mesures (Annals of Allergy, vol. 41, sept. 1978, p. 164—169) concernant toute une série d'enzymes, telles que phosphatases, estérases, lipases, leucine-amino-peptidase, valine-amino-peptidase, trypsine, chymotrypsine, béta-glucose-aminidase, des glucosidases, etc.

En appliquant le système API ZYM aux produits des figures 1 et 2, ci-dessus décrites, on a trouvé les résultats rapportés dans le tableau 1 qui suit. Ce tableau donne pour les 20 cupules du système API la note (de 0 à 5) dèterminée par comparaison de l'échelle colorée du système avec la teinte développée dans la cupule. Les lettres "tr" signifient "trace". Les essais sont bien entendu accompagnés d'un échantillon témoin formé par une solution de phosphate disodique à 25 g/l contenant du merthiolate, et un extrait chauffé de pollen.

Il résulte des données du tableau 1 que l'extrait brut contient nettement des enzymes et que la teneur en cellesci est un peu diminuée du fait de la purification au sulfate d'ammonium. Les enzymes disparaissent pratiquement tout à fait à la suite du fractionnement par filtration sur gel; il n'en reste pratiquement plus à partir de la 22 ème fraction; on en trouve, par contre, dans les fractions 14 à 18 dépourvues d'activité allergénique. (Voir Tableau 1, à la page 8).

On a signalé plus haut que l'élimination des composants de masses moléculaires inférieures à 14 000 et supérieures à 45 000 améliore également l'adsorption de l'allergène par des gels minéraux. Ainsi peut-on constater que les fractions utiles, séparées suivant la figure 2, s'adsorbent mieux sur les adsorbants connus, tels que par exemple alumine, phosphate d'alumine ou phosphate de calcium. L'adsorption est particulièrement efficace pour le phosphate spécial dans lequel le rapport pondéral Ca/P est compris entre 1,55 et 1,90, comme décrit dans le brevet français n° 72 12036 (publication 2 181 426 du 7.12.1973).

Un tel gel est préparé notamment par mélange d'une solution de 25 g de phosphate disodique dans 1 litre d'eau, avec 2/10 000 de merthiolate, additionnée de 20 ml de l'extrait allergénique, préparé à partir de 100 g de pollen, comme indiqué au début de la présente description. Au mélange obtenu, on ajoute 1 litre d'eau renfermant 10,2 g de $CaCl_2.2H_2O$. Cette addition est effectuée très rapidement sous agitation et le pH du milieu est amené à 6,8—7 au moyen de la soude normale.

Dans une première série d'essais, on a déterminé, par la méthode de la piqûre mentionnée plus haut, la réaction sur la peau de 11 patients. Pour chacun d'eux, on a utilisé un extrait de phléole purifié par précipitation au sulfate d'ammonium et — d'autre part — le liquide surnageant après la précipitation du phosphate de calcium spécial en présence du même extrait, comme on vient de l'indiquer. Dans le deux cas, la dilution de l'extrait est de 1/1000. Les résultats sont encore indiquées au moyen de + dont chacun correspond à 5 mm de papule formée sur la peau du patient.

(Voir tableau 2, à la page 7).

On voit qui l'adsorption a été très efficace, puisque la diminution de la réaction sur la peau globale a été de

$$\frac{(22—8,5)}{22} = 61,4\%$$

Des essais similaires ont été effectués avec le même phosphate de calcium spécial mélangé non plus avec l'extrait total de phléole, mais avec les fractions dépourvues d'enzymes, représentées et décrites à propos de la figure 2. Les réactions sur la peau ont été déterminées à des dilutions allant de 1/1 000 à 1/1 000 000, le tableau 3 en donne les résultats comparés d'une part, pour l'extrait, c'est-à-dire les fractions elles-mêmes, et, d'autre part, sur le liquide surnageant après la précipitation du phosphate. Pour la dilution de 1/1 000, la réaction est déterminée par la méthode de la piqûre, tandis qu'elle l'est par intradermoréaction, pour les autres dilutions.

(Voir tableau 3, à la page 11).

On voit que l'adsorption de l'extrait de phléole fractionné est très poussée.

Des résultants similaires sont obtenus avec des extraits obtenus à partir d'autres pollens, notamment ceux du seigle, de l'ivraie, de dactyle, etc.

TABLEAU 1

| Echantillon | Concentration | Numéros des cupules | | | | | | | | | | | | | | | | | | | |
|---|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | 18 | 19 | 20 |
| Témoin | | | | tr | tr | | | | | | | | tr | | | | | | | | |
| Extrait brut, total (fig. 1) | 1/100 | | 5 | ~5 | 3 | tr | 4 | 3 | 3 | 3 | tr | >5 | >5 | 2 | | | | | | 3 | 1 | |
| Extrait purifié au sulf. d'$NH_4$ total (fig. 2) | 1/100 | | 4 | 3 | 2 | | 3 | | 1 | | | 5 | 5 | | | | | | | | 1 | |
| d° fractions 14 à 18 | 1/34,5 | | ≤1 | 3 | 1 | | 3 | | | | | 5 | 4 | | | | | | | | tr | |
| d° fraction 15 | 1/100 | | | 1 | tr | | | | | | | 5 | 2 | | | | | | | | tr | |
| d° fraction 22 | 1/100 | | | 1 | tr | | | | | | | 1 | 1 | | | | | | | | | |
| d° fraction 24 | 1/100 | | | 0,5 | tr | | | | | | | tr | 1 | | | | | | | | | |
| d° fraction 26 | 1/100 | | | tr | tr | | | | | | | | 1 | | | | | | | | | |
| d° fraction 29 | 1/100 | | | tr | tr | | | | | | | | 1 | | | | | | | | | |
| d° fraction 31 | 1/100 | | | tr | tr | | | | | | | | 1 | | | | | | | | | |
| d° fraction 31 | 1/7 | | | 1 | ≤1 | | | | | | | | tr | | | | | | | | | |
| d° fraction 51 | 1/100 | | | tr | tr | | | | | | | | 1 | | | | | | | | | |
| d° fraction 56 | 1/100 | | | tr | tr | | | | | | | | 1 | | | | | | | | | |

TABLEAU 2

| nº patient | 1 | 2 | 3 | 4 | 5 | 6 | 7 | 8 | 9 | 10 | 11 | Total |
|---|---|---|---|---|---|---|---|---|---|---|---|---|
| extrait | ++ | + | +++ | +± | +++ | +± | + | +++ + | + | ++ | ++ | 22 |
| surnageant | + | 0 | + | ± | ++ | ± | ± | + | 0 | +± | ± | 8,5 |

TABLEAU 3

DILUTIONS

| Patient nº | Extrait liquide | | | | Surnageant | | | |
|---|---|---|---|---|---|---|---|---|
| | $1/10^6$ | $1/10^5$ | $1/10^4$ | $1/10^3$ | $1/10^6$ | $1/10^5$ | $1/10^4$ | $1/10^3$ |
| 1 | +± | ++ | | | ± | ± | | |
| 2 | | | | ++ | | | | + |
| 3 | ± | + | ++± | | 0 | 0 | +± | |
| 4 | +++ | ++++ | | | + | +± | | |
| 5 | + | + | +± | 0 | 0 | 0 | 0 | 0 |
| 6 | 0 | 0 | | 0 | 0 | 0 | | 0 |
| 7 | ++ | +++ | | + | 0 | 0 | | + |
| 8 | ++ | | | ± | + | | | ± |
| 9 | ± | ++± | +++ | 0 | 0 | + | ++ | 0 |
| 10 | + | ++± | | 0 | ± | ± | | 0 |
| 11 | | | | + | | | | 0 |
| 12 | +± | +± | | ± | ± | ± | | 0 |
| 13 | +± | ++ | | + | ± | ± | | 0 |
| 14 | + | ++ | | 0 | + | ± | | 0 |
| Total | 15,5 | 21,5 | 7 | 6 | 5 | 5 | 3,5 | 2,5 |
| Diminution % | | | | | 67,7 | 76,7 | 50 | 58,3 |

**Revendications**

1. Composition allergénique, aqueuse, dépourvue d'enzymes, obtenue par la macération d'une poudre de pollen, de farine, de poussière de maison, de kapok, de laine ou de moisissures, dans un milieu aqueux, suivie de la séparation de la poudre, de la précipitation des protéines à partir du liquide ainsi séparé et leur redissolution, de façon à former une solution aqueuse, qui est ensuite fractionnée chromatographie, caractérisée en ce qu'elle renferme toutes les protéines allergéniquement actives de ladite solution, de masses moléculaires de 10 000 à 55 000, à l'exclusion de tout composé organique de masse moléculaire inférieure à 10 000 ou supérieure à 55 000.

2. Composition suivant la revendication 1, caractérisée en ce qu'elle renferme toutes les protéines allergéniquement actives, de ladite solution, dont les masses moléculaires sont de 14 000 à 45 000, à l'exclusion de tous composés organiques de masses moléculaires situées en dehors de ces limites.

3. Composition suivant la revendication 1, caractérisée en ce qu'elle renferme toutes les protéines allergéniquement actives, de ladite solution, dont les masses moléculaires sont de 20 000 à 44 000, à l'exclusion de tous composés organiques de masses moléculaires situées en dehors de ces limites.

4. Composition suivant l'une des revendications 1 à 3, caractérisée en ce que tous ses composants des masses moléculaires indiquées se trouvent à l'état adsorbé sur un gel aqueux, minéral.

5. Composition suivant la revendication 4, caractérisée en ce que le gel aqueux est un gel d'alumine, de phosphate d'aluminum ou de phosphate de calcium.

6. Composition suivant l'une des revendications 4 ou 5, caractérisée en ce que toutes ses protéines des masses moléculaires indiquées sont adsorbées sur un gel aqueux de phosphate de calcium, dans lequel le rapport pondéral Ca/P est de 1,55 à 1,90 et, de préférence, 1,62 à 1,85.

7. Procédé pour la préparation d'une composition allergénique aqueuse, suivant la revendication 1, selon lequel la solution aqueuse, séparée du solide dont on a extrait l'allergène, est purifié, caractérisé en ce que l'on soumet la solution à un fractionnement chromatographique de façon à éliminer toutes les substances de masses moléculaires inférieures à 10 000 et supérieures à 55 000, initialement présentes dans la solution, tandis que toutes les fractions intermédiares sont recueillies.

8. Procédé suivant la revendication 7, caractérisée en ce que l'élimination des substances non actives est effectuée par tamisage moléculaire.

9. Procédé suivant la revendication 7 ou 8, caractérisé en ce que seulement les protéines de masses moléculaires de 20 000 à 44 000 sont retenues pour constituer la composition allergénique.


**Patentansprüche**

1. Wässrige enzymlose allergene Zusammensetzung, erhalten durch Zerkleinern eines Pulvers aus Blütenstaub, Mehl, Hausstaub, Kapok, Wolle oder Schimmel in einem wässrigen Medium mit nachfolgendem Abtrennen des Pulvers, Ausfällen der Proteine aus der dabei abgetrennten Flüssigkeit und deren Wiederauflösen zur Bildung einer wässrigen Lösung, die anschließend chromatographisch fraktioniert wird, dadurch gekennzeichnet, daß sie alle aktiv allergenen Proteine der Lösung mit Molekulargewichten von 10 000 bis 55 000 unter Ausschluß aller organischen Verbindungen mit Molekulargewichten von weniger als 10 000 oder mehr als 55 000 enthält.

2. Zusammensetzung nach Anspruch 1, dadurch gekennzeichnet, daß sie alle aktiv allergenen Proteine der Lösung, deren Molekulargewichte 14 000 bis 55 000 betragen, unter Ausschluß aller oranischen Verbindungen, deren Molekulargewichte außerhalb dieser Grenzen liegen, enthält.

3. Zusammensetzung nach Anspruch 1, dadurch gekennzeichnet, daß sie alle aktiv allergenen Proteine der Lösung, deren Molekulargewichte 20 000 bis 44 000 betragen, unter Ausschluß aller organischen Verbindungen, deren Molekulargewichte außerhalb dieser Grenzen liegen, enthält.

4. Zusammensetzung nach einem der Ansprüche 1 bis 3, dadurch gekennzeichnet, daß alle ihre Bestandteile der angegebenen Molekulargewichte sich im adsorbierten Zustand auf einem wässrigen anorganischen Gel befinden.

5. Zusammensetzung nach Anspruch 4, dadurch gekennzeichnet, daß das wässrige Gel ein Aluminiumoxidgel, ein Aluminiumphosphatgel oder eine Calciumphosphatgel ist.

6. Zusammensetzung nach einem der Ansprüche 4 oder 5, dadurch gekennzeichnet, daß alle ihre Proteine der angegebenen Molekulargewichte auf einem wässrigen Calciumphosphatgel adsorbiert sind, in dem das Gewichtsverhältnis Ca/P 1,55 bis 1,90 und vorzugsweise 1,62 bis 1,85 beträgt.

7. Verfahren zur Herstellung einer wässrigen allergenen Zusammensetzung des Anspruchs 1, bei dem die von dem Festkörper, aus dem man das Allergen extrahiert hat, abgetrennte wässrige Lösung gereinigt wird, dadurch gekennzeichnet, daß man die Lösung einer chromatographischen Fraktionierung zum Entfernen aller Substanzen der Molekulargewichte von weniger als 10 000 und mehr als 55 000 unterzieht, die anfänglich in der Lösung vorhanden waren, während alle dazwischenliegenden Fraktionen rückgewonnen werden.

8. Verfahren nach Anspruch 7, dadurch gekennzeichnet, daß das Entfernen der nichtaktiven Substanzen durch Molekularsieben durchgeführt wird.

9. Verfahren nach Anspruch 7 oder 8, dadurch gekennzeichnet, daß nur die Proteine der Molekulargewichte von 20 000 bis 44 000 zur Bildung der allergenen Zusammensetzung zurückgehalten werden.


**Claims**

1. Allergenic aqueous composition, free from enzymes, obtained by steeping a powder of pollen, flour, house dust, kapok, wool or molds in an aqueous medium, followed by the separation of the powder, the precipitation of proteins from the liquid thus separated and their redissolving to form an aqueous solution which is then fractionated by chromatography, characterized in that it contains all the allergenically active proteins of molecular masses 10 000 to 55 000 of said solution, exclusive of any organic compound of molecular mass below 10 000 or above 55 000.

2. Composition according to claim 1, characterized in that it contains all the allergenically active proteins of said solution, the molecular masses of which are 14 000 to 44 000, to the exclusion of all organic compounds of molecular masses falling beyond these limits.

3. Composition according to claim 1, characterized in that it contains all the allergenically active proteins of said solution, the molecular masses of which are 20 000 to 44 000, to the exclusion of all organic compounds of molecular masses falling beyond these limits.

4. Composition according to one of claims 1 to 3, characterized in that all its components having the set forth molecular masses are adsorbed on a mineral aqueous gel.

5. Composition according to claim 4, characterized in that the aqueous gel is a gel of alumina, of aluminium phosphate or of calcium phosphate.

6. Composition according to one of claims 4 or 5, characterized in that all its proteins having the indicated molecular masses are adsorbed on an aqueous gel of calcium phosphate in which the weight ratio Ca/P is 1.55 to 1.90 and preferably 1.62 to 1.85.

7. Process for the preparation of an allergenic aqueous composition, according to claim 1, wherein an aqueous solution, separated from the solid from which allergen has been extracted, is purified, characterized in that the solution is subjected to a chromatographic fractionation so as to eliminate all the substances having molecular masses below 10 000 and above 55 000, which were initially present in the solution, while all intermediate fractions are recovered.

8. Process according to claim 7, characterized in that the elimination of the non active substances is carried out by molecular screening.

9. Process according to claim 7 or 8, characterized in that only proteins having molecular masses of 20 000 to 44 000 are recovered to constitute the allergenic composition.

Fig.1

Absorbance à 280 nm

0,2

0,1

O          ++          ++          +          O    O    O
                        ±
A          B    C              D    E          F

10  12  14  16  18  20  22  24  26  28  30  32  34  36  38  40  42  44  46  48  50  52  54  56

Fraction N°

Fig. 2